# EUROPÄISCHE PATENTSCHRIFT

(11) **EP 2 018 889 B1**
(45) Veröffentlichungstag und Bekanntmachung des Hinweises auf die Patenterteilung: **17.10.2012**
(21) Anmeldenummer: 08156418.9
(22) Anmeldetag: 19.05.2008
(51) Int. Cl.: A61Q 5/02, A61Q 19/10, A61K 8/39, A61K 8/44, A61K 8/46

(54) **Wässrige tensidische Formulierung enthaltend Polypropylenglykol(3)myristylether**
Aqueous surfactant-containing formulation containing polypropylenglycol(3)myristyl ether
Formule tensioactive aqueuse contenant du polypropylenglycol(3)myristyl éther

(30) Priorität: 20.07.2007 DE 102007034438
(43) Veröffentlichungstag der Anmeldung: 28.01.2009
(73) Patentinhaber: Evonik Goldschmidt GmbH, 45127 Essen (DE)
(72) Erfinder: Leidreiter, Holger, 45529, Hattingen (DE); Kortemeier, Uta, 40217 Düsseldorf (DE)

(56) Entgegenhaltungen:
- EP-A- 0 365 160
- EP-A- 0 615 748
- EP-A- 1 316 307
- WO-A-01/56537
- WO-A-99/13857
- US-A1- 2004 115 155

## Beschreibung

Die vorliegende Erfindung bezieht sich auf wässrige tensidische Formulierungen, insbesondere flüssige Körperreinigungsmittel, wie zum Beispiel Duschbäder und Haarshampoos. Sie bezieht sich weiterhin auf die Verwendung von Polypropylenglykol(3)myristylether als vorzugsweise alleiniger Viskositätsregulator, Pflegewirkstoff und Solubilisator in wässrigen tensidischen Formulierungen, wie den genannten Duschbädern und Haarshampoos.

Moderne kosmetische Reinigungsprodukte für Haut und Haar, wie beispielsweise Duschbäder und Haarshampoos, bestehen im wesentlichen aus
- Wasser als wichtigstem Lösemittel,
- Tensiden,
- Viskositätsregulatoren zum Verdicken der Formulierung,
- Lösungsvermittlern (Solubilisatoren) für wasserunlösliche Substanzen,
- Parfümölen,
- Konservierungsstoffen sowie
- Wirkstoffen zur Pflege von Haut und Haaren, wie z.B. Rückfettern.

Typische Tenside in Körperreinigungsmitteln sind anionischer, amphoterer und zwitterionischer Struktur. Zu den anionischen Tensiden zählen insbesondere die Salze verschiedener Kationen (Natrium, Ammonium oder andere) von Laurylsulfat, Laurylethersulfat, Myristylethersulfat. Als zwitterionische Tenside werden u.a. Cocamidopropylbetain oder Cocoamidopropylsultain eingesetzt. Amphotere Tenside sind insbesondere Amphoacetate wie Natriumcocoamphoacetat oder Dinatriumcocoamphodiacetat.

Typische, nach dem bisherigen Stand der Technik eingesetzte Verdicker sind NaCl, niedermolekulare nichtionische Tenside, wie Cocosnußfettsäuremonoethanolamid/-diethanolamid und Laureth-3, oder polymere, hochmolekulare, assoziative, hochethoxylierte Fettderivate, wie z.B. Polyethylenglykol(9000)-hydriertes Glycerinpalmitat.

Als Solubilisator oder auch Lösungsvermittler wird im Sinne der vorliegenden Erfindung eine Substanz bezeichnet, die in der Lage ist, wasserunlösliche Verbindungen möglichst klar in wässrigen Systemen in Lösung zu bringen. Nach allgemein akzeptierter Vorstellung werden bei diesem Vorgang Mizellen gebildet, in deren flüssigkristalliner Struktur die hydrophoben Substanzen integriert sind. Optimal ist die Bildung einer "Mikroemulsion", also einer thermodynamisch stabilen Mischung aus Wasser (wässrige Lösung), einem Öl (nicht mit Wasser mischbare Substanz) und einem Solubilisator/Lösungsvermittler. Typische Solubilisatoren sind ethoxylierte Fettderivate.

Parfümöle werden den Formulierungen allgemein zur Verbesserung der olfaktorischen Eigenschaften zugesetzt. Die Akzeptanz durch den Verbraucher spielt hier die wichtigste Rolle. Daneben ist es möglicherweise vorteilhaft, die Eigengerüche verwendeter Rohstoffe mit Parfümölen zu überdecken.

Konservierungsstoffe werden zur mikrobiologischen Stabilisierung eingesetzt. Im Falle einer Kontamination sollen diese Inhaltstoffe das Keimwachstum verhindern und gegebenenfalls auch Keime abtöten. Konservierungsmittel sind in behördlichen Regularien (z.B. EU-Kosmetikverordnung) ausführlich beschrieben und reguliert.

Typische Pflegeadditive sind ethoxylierte Glycerin-Fettsäureester, wie beispielweise Polyethylenglykol(7)glycerylmonococoat, oder kationische Polymere, wie beispielsweise Polyquaternium-7.

Bei der Hautreinigung werden neben dem lipophilen Schmutz auch hauteigene Lipide durch die verwendeten Tenside abgewaschen. Dieser Effekt wird oft als unangenehm empfunden, die Haut fühlt sich rau und spröde an. Die Haut wird auch als "trocken" bezeichnet, wobei hier jedoch die Abwesenheit von Fett gemeint ist.
Sogenannte Rückfettungsmittel werden Körperreinigungsmitteln zugesetzt, damit der beschriebene Entfettungsvorgang vermindert wird. Im Ergebnis kann einerseits das abgewaschene Fett durch das Rückfettungsmittel ersetzt werden, andererseits aber auch die entfettende Wirkung der Formulierung an sich durch den Einsatz des Rückfetters vermindert werden.

Formulierungstechnisch ist es schwierig, Emollients (kosmetische Öle), wie z.B. Isopropylmyristat (TEGOSOFT M^{®}), zu diesem Zweck einzusetzen, weil diese Öle aufwendig solubilisiert werden müssen. Gebräuchlichere Rückfetter sind daher hydrophilere Produkte, wie z.B. Polyethylenglykol(7)glycerylmonococoat (TEGOSOFT GC^{®}), die bereits durch den Überschuss der reinigenden Tenside solubilisiert werden. Die Analyse einer Produktdatenbank, welche weltweite Produktinnovationen in Verbrauchermärkten erfasst ("Global New Products Database": Mintel), ergab, dass 29 % aller Hautreinigungsformulierungen im europäischen Markt im Untersuchungszeitraum (9/05 - 9/06) Polyethylenglykol(7)glycerylmonococoat enthielten.

Es wird angenommen, dass der Rückfettungsvorgang beim Abspülen der Formulierung nach der eigentlichen Wäsche stattfindet. Beim Abwaschvorgang mit Wasser wird die vorhandene Lösung soweit verdünnt, bis die sog. CMC (kritische Mizellbildungskonzentration) unterschritten ist. Mit der Freisetzung der Mizellkomponenten (die lipophilen Rückfetter, die Tenside und Solubilisatoren) werden die Rückfetter wieder unlöslich. Diese lipophilen Substanzen (sowohl hauteigene Lipide als auch Emollients/kosmetische Öle) fallen aus und ziehen auf die Haut auf.

Zu den Anforderungen an die fertige Formulierung gehören also neben der reinigenden Wirkung ein cremiger Schaum, ein Schutz vor dem Austrocknen der Haut und eine gute Pflegeleistung. Zu den Grundanforderungen an die einzelnen Bestandteile gehören eine gute Hautverträglichkeit und Verarbeitbarkeit. Es wäre deshalb vorteilhaft, wenn möglichst viele der Anforderungen an eine kosmetische Formulierung durch möglichst wenige toxikologisch unbedenkliche und universell einsetzbare Bestandteile erfüllt werden könnten.

Die DE-A 40095533 beschreibt schaumgedämpfte nichtionische Tensidgemische enthaltend 20 bis 98 Gew.-% eines Alkylpolyglykosids sowie 80 bis 2 Gew.-% eines Polyglykolethers. Letztere Komponente wird dem Tensidgemisch wegen ihrer schaumdämpfenden Wirkung zugesetzt. Zu den bevorzugt eingesetzten Polyglykolethern gehören auch Alkylether propoxylierter Fettalkohole. Polypropylenglykol(3)myristylether wird jedoch in der gesamten Druckschrift nicht explizit genannt. Auch enthalten die mit den offenbarten Tensidsystemen hergestellten flüssigen Waschmittel eine Vielzahl zusätzlicher Komponenten, und es finden sich keinerlei Hinweise dafür, dass die eingesetzten Polyglykolether neben ihrer schaumdämpfenden Wirkung noch weitere Funktionen in der jeweiligen Waschmittel-Formulierung erfüllen. Weiterhin unterscheiden sich die beispielhaft genannten Waschmittelsysteme von den erfindungsgemäßen Formulierungen durch ihren mit ca. 35 Gew.-% vergleichsweise niedrigen Wassergehalt.

Die EP-A 0459769 offenbart Reinigungsmittelzusammensetzungen, insbesondere Seifenstücke, welche verminderte Aufweichung sowie erhöhte Sanftheit zeigen. Die beschriebenen Zusammensetzungen enthalten Alkalisalze höherer Fettsäuren zu einem Anteil größer als 25 Gew.-%, 1-50 Gew.-% eines weiteren Tensids, 1-15 Gew.-% einer freien Fettsäure sowie 1-15 Gew.-% einer die Aufweichung vermindernden Komponente. Zu diesen Komponenten werden auch die Alkylether alkoxylierter Fettalkohole, wie beispielsweise Polypropylenglykol(3)myristylether, gezählt. Weitere Wirkungsweisen werden jedoch nicht genannt. Auch ist offensichtlich, dass sich die offenbarten Reinigungsmittelzusammensetzungen aufgrund ihres sehr niedrigen Wassergehaltes (ca. 10 Gew.-%) deutlich von den erfindungsgemäßen Zusammensetzungen unterscheiden.

In der EP-A 1407761 werden wässrige alkoholische Zusammensetzungen, beispielsweise Desinfektionslösungen, beschrieben, die Alkohol und Wasser in einem Gewichtsverhältnis von 60:40 bis 100:0 sowie zwischen 0,5 und 8 Gew.-% eines Verdickersystems umfassend mindestens einen Emulgator enthalten. Eine Vielzahl weiterer Bestandteile können in den wässrig alkoholischen Zusammensetzungen vorhanden sein, darunter auch Emollients, wie Polypropylenglykol(3)myristylether. Zwar findet sich in der Schrift der Hinweis, dass, obwohl ein Verdickersystem für die Stabilität und Konsistenz der beschriebenen Zusammensetzungen verantwortlich sei, auch Emollients die Viskosität und Stabilität der Zusammensetzungen beeinflussen könnten, jedoch kann ein Effekt aus den experimentellen Daten nicht abgeleitet werden. Auch darf bei der Bewertung der beschriebenen Effekte der teilweise recht hohe Alkoholgehalt der untersuchten Systeme nicht außer Acht gelassen werden.

Die US-A 7087221 schließlich offenbart wässrige Shampoo-Zusammensetzungen mit verbesserten Haar konditionierenden Eigenschaften enthaltend mindestens 20 Gew.-% Wasser, zwischen 5 und 50 Gew.-% eines Tensids und mindestens 0,05 Gew.-% eines Polyalkylenoxidalkylethers, der einen HLB-Wert (hydrophilic-lipophilic-balance) kleiner 10 haben soll. Polypropylenglykol(3)myristylether befindet sich nicht unter den beispielhaft genannten Verbindungen. Die große Anzahl möglicher weiterer Komponenten sowie die komplexen Testrezepturen verdeutlichen, dass sich auch in diesem Dokument keine Lehre findet, die mit der der vorliegenden Erfindung zugrunde liegenden Aufgabe in Verbindung gebracht werden könnte.

Keine der genannten Schriften offenbart also die Verwendung von Emollients, insbesondere Polypropylenglykol(3)myristylether, als Viskositätsregulator, Pflegewirkstoff oder Solubilisator in wässrigen tensidischen Formulierungen.

Aufgabe der vorliegenden Erfindung ist es, eine Substanz zu finden, die nicht nur als alleiniger Viskositätsregulator eingesetzt werden kann, sondern darüber hinaus weitere Eigenschaften wie gute Pflegeleistung (Rückfettung), gute Schaumeigenschaften und hohes Solubilisierungsvermögen mitbringt, so dass auf weitere Viskositätsregulatoren und gegebenenfalls auch auf weitere Rückfetter und Solubilisatoren in der jeweils gewählten Formulierung verzichtet werden kann.

Überraschenderweise wurde gefunden, dass sich Polypropylenglykol(3)myristylether nicht nur in wässrigen tensidischen Formulierungen, d.h. tensidischen Formulierungen mit einem Wassergehalt von mindestens 75 Gew.-%, ohne weitere Hilfsmittel löst, sondern darüber hinaus auch noch die Viskosität der Formulierung erhöht, die Schaumcremigkeit und das Hautgefühl während der Anwendung verbessert und solubilisierende Eigenschaften für wasserunlösliche Substanzen besitzt.

Gegenstand der vorliegenden Erfindung ist somit die Verwendung von Polypropylenglykol(3)myristylether als vorzugsweise alleiniger Viskositätsregler, Pflegewirkstoff und Solubilisator in wässrigen tensidischen Formulierungen mit einem Wassergehalt von mindestens 75 Gew.-%.

Ein weiterer Gegenstand der vorliegenden Erfindung sind wässrige tensidische Formulierungen enthaltend oder bestehend aus mindestens 2 Gew.-% Polypropylenglykol(3)myristylether, mindestens 10 Gew.-% mindestens eines Tensids und mindestens 85 Gew.-% Wasser.

Ein weiterer Gegenstand der vorliegenden Erfindung sind flüssige Körperreinigungsmittel, insbesondere Duschbäder und Haarshampoos, enthaltend eine solche wässrige tensidische Formulierung.

Erfindungsgemäß bevorzugt verwendete Tenside sind die Verbindungen Natriumlaurylethersulfat und Cocamidopropylbetain sowie Kombinationen davon. Daneben können aber auch alle bekannten Tenside in den erfindungsgemäßen Zusammensetzungen verwendet werden. Weiterhin können die erfindungsgemäßen Zusammensetzungen die allgemein üblichen Hilfs- und Zusatzstoffe enthalten, z.B. Viskositätsregulatoren, Lösungsvermittler (Solubilisatoren), Parfümöle, Konservierungsstoffe sowie Wirkstoffe zur Pflege von Haut und Haaren, wie z.B. Rückfetter. Erfindungsgemäß bevorzugt wird Polypropylenglykol(3)myristylether jedoch als alleiniger Viskositätsregulator, Rückfetter und Solubilisator eingesetzt.

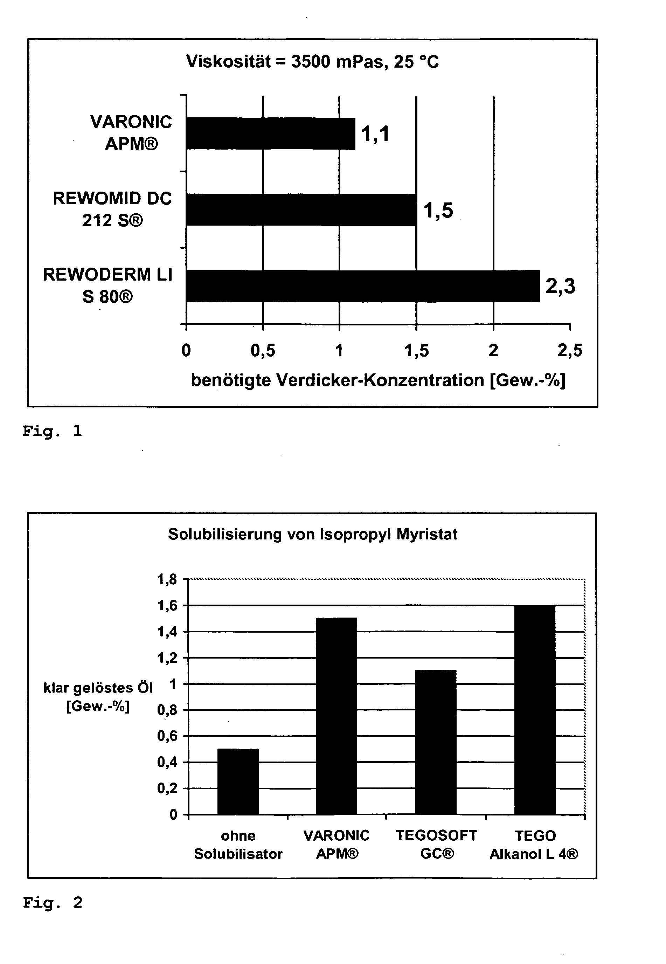

Die vorliegende Erfindung wird durch die Figuren Fig. 1 und Fig. 2 näher erläutert, ohne dass die Erfindung auf die dort dargestellte Ausführungsform beschränkt sein soll. In Fig. 1 sind die Ergebnisse der Beispiele 1 zur Verdickungswirkung von Polypropylenglykol(3)myristylether graphisch dargestellt. In Fig. 2 sind die Ergebnisse der Solubilisierungsversuche gemäß den Beispielen 3 graphisch wiedergegeben.

Die folgenden Beispiele dienen der Veranschaulichung der vorliegenden Erfindung, stellen jedoch keinerlei Einschränkung des Schutzumfangs dar.

### Beispiele 1a-c: Verdickung

Die verdickende Wirkung von Polypropylenglykol(3)myristylether (VARONIC APM^{®}) wurde im Vergleich zu zwei gängigen tensidischen Verdickern (Cocamide DEA (REWOMID DC 212 S^{®}) und Polyethylenglykol(9000) hydriertes Glycerinpalmitat/ Polyethylenglykol(7)glycerylmonococoat (REWODERM LI S 80^{®})) getestet. Ein standardisiertes Tensidsystem, bestehend aus 9 Gew.-% Natriumlaurylethersulfat (Texapon NSO^{®}), 3 Gew.-% Cocamidopropylbetain (TEGO Betain F 50^{®}) und 0,7 Gew.-% NaCl, wurde auf eine Viskosität von 3500 mPas bei 25 °C eingestellt. Die dazu jeweils benötigte Verdickerkonzentration ist in Tabelle 1 sowie in Fig. 1 dargestellt. Es zeigt sich, dass Polypropylenglykol(3)myristylether (VARONIC APM^{®}) am effektivsten ist, da die geringste Einsatzkonzentration benötigt wird.

**Tabelle 1: Verdickungswirkung von Polypropylenglykol(3)myristylether.**

| | Bsp. 1a* | Bsp. 1b* | Bsp. 1c* |
|---|---|---|---|
| | Gew.-% | Gew.-% | Gew.-% |
| Texapon NSO^{®} (INCI: Sodium Laureth Sulfate) | 9,0 | 9,0 | 9,0 |
| TEGO Betain F 50^{®} (INCI: Cocamidopropyl Betaine) | 3,0 | 3,0 | 3,0 |
| VARONIC APM^{®} (INCI: Polypropylenglykol(3)myristyl-ether) | 1,1 | | |
| REWOMID DC 212 S^{®} (INCI: Cocamide DEA) | | 1,5 | |
| REWODERM LI S 80^{®} (INCI: PEG-200 Hydrogenated Glyceryl Palmate/ PEG-7 Glyceryl Cocoate) | | | 2,3 |
| NaCl | 0,7 | 0,7 | 0,7 |
| Wasser, demineralisiert | ad 100,0 | | |
| Viskosität [mPas], gemessen bei 25 °C mit einem Brookfield LVF, Spindel 3, 5 Upm | 3500 | | |

| | | | |
|---|---|---|---|
| * Vergleichsbeispiel | | | |

### Beispiele 2a-g: Hautpflegeleistung und Schaumeigenschaften

Zur Bewertung der Pflegeleistung und der Schaumeigenschaften von Polypropylenglykol(3)myristylether (VARONIC APM^{®}) wurden sensorische Handwaschtests im Vergleich zu Polyethylenglykol-(7)glycerylmonococoat (TEGOSOFT GC^{®}) durchgeführt.

Polyethylenglykol(7)glycerylmonococoat gilt als Markstandard für hydrophile Emollients/Rückfetter. Eine Gruppe bestehend aus 10 trainierten Prüfpersonen wusch sich dabei definiert die Hände und bewertete Schaumeingeschaften, Hautgefühl und Abspülverhalten anhand einer Notenskala von 1 (schlecht) bis 5 (sehr gut). Die eingesetzten Produkte wurden jeweils in zwei standardisierten Tensidformulierungen getestet. Testreihe 1 wurde mit Tensidsystem A (Natriumlaurylethersulfat/Cocamidopropylbetain), Testreihe 2 wurde mit Tensidsystem B (Natriumlaurylethersulfat) durchgeführt. Da Cocamidopropylbetain (TEGO Betain F 50^{®}) bereits hautkonditionierende Eigenschaften hat, wurde in Testreihe 2 nur das Aniontensid Natriumlaurylethersulfat (Texapon NSO^{®}) mit den Emollients kombiniert. Die sensorischen Testergebnisse sind in den Tabellen 3 und 4 zusammengefasst.

**Tabelle 2 : Formulierungen für Handwaschtest**

| | Testreihe 1 (Tensidsystem A) | | | | Testreihe 2 (Tensidsystem B) | | |
|---|---|---|---|---|---|---|---|
| | Bsp. 2a* | Bsp. 2b | Bsp. 2c* | Bsp. 2d | Bsp. 2e* | Bsp. 2f* | Bsp. 2g |
| | Gew.-% | Gew.-% | Gew.-% | Gew-% | Gew.-% | Gew.-% | Gew.-% |
| Texapon NSO^{®} (INCI: Sodium Laureth Sulfate) | 9,0 | 9,0 | 9,0 | 9,0 | 12,0 | 12,0 | 12,0 |
| TEGO Betain F 50^{®} (INCI: Cocamidopropyl Betaine) | 3,0 | 3,0 | 3,0 | 3,0 | | | |
| VARONIC APM^{®} (INCI: Polypropylenglykol(3)myristylether) | 1,0 | 2,0 | | | 1,0 | | |
| TEGOSOFT GC^{®} (INCI: PEG-7 Glyceryl Cocoate) | | | 2,0 | | | 1,0 | |
| Wasser | ad 100,0 | | | | | | |

| | | | | | | | |
|---|---|---|---|---|---|---|---|
| * Vergleichsbeispiel | | | | | | | |

**Tabelle 3: Sensorische Ergebnisse - Handwaschtest mit Tensidsystem A**

| | Bsp. 2c* | Bsp. 2a* | Bsp. 2b | Bsp. 2d (Control) |
|---|---|---|---|---|
| Anschäumverhalten | 3,2 | 3,1 | 3,2 | 3,4 |
| Schaumvolumen | 2,9 | 3,0 | 3,2 | 3,3 |
| Schaumcremigkeit | 2,6 | 3,0 | 2,5 | 2,9 |
| Hautgefühl während des Waschens | 3,3 | 4,0 | 3,8 | 3,6 |
| Abspülbarkeit | 4,1 | 4,4 | 4,3 | 4,5 |
| Hautglätte | 2,4 | 2,5 | 2,5 | 2,3 |
| Hautweichheit | 2,7 | 2,7 | 2,7 | 2,6 |
| Hautglätte nach 3 min. | 3,5 | 3,5 | 3,4 | 3,5 |
| Hautweichheit nach 3 min. | 3,5 | 3,5 | 3,6 | 3,3 |

| | | | | |
|---|---|---|---|---|
| * Vergleichsbeispiel | | | | |

In Tabelle 3 sind die Ergebnisse der 1. Testreihe dargestellt. Üblicherweise reduziert der Zusatz eines Emollients die Schaumquantität, erhöht aber die Schaumqualität und verbessert das Hautgefühl. Diese erwünschten Eigenschaften wurden auch für Polypropylenglykol(3)myristylether (VARONIC APM^{®}) gefunden, teilweise deutlich ausgeprägter als für den Marktstandard Polyethylenglykol(7)glycerylmonococoat (TEGOSOFT GC^{®}).

**Tabelle 4: Sensorische Ergebnisse - Handwaschtest mit Tensidsystem B**

| | Bsp. 2f* | Bsp. 2e* | Bsp. 2g (Control) |
|---|---|---|---|
| Anschäumverhalten | 3,6 | 3,6 | 3,6 |
| Schaumvolumen | 3,5 | 3,3 | 3,5 |
| Schaumcremigkeit | 2,9 | 2,8 | 2,6 |
| Hautgefühl während des Waschens | 3,5 | 3,6 | 3,5 |
| Abspülbarkeit | 4,1 | 4,1 | 4 |
| Hautglätte | 2,3 | 2,6 | 2,2 |
| Hautweichheit | 2,6 | 2,9 | 2,6 |
| Hautglätte nach 3 min. | 3,5 | 3,4 | 3,2 |
| Hautweichheit nach 3 min. | 3,4 | 3,3 | 3 |

| | | | |
|---|---|---|---|
| * Vergleichsbeispiel | | | |

In Tabelle 4 sind die Ergebnisse der 2. Testreihe dargestellt. Der alleinige Einsatz des Aniontensids führt zu einer geringeren Differenzierbarkeit bei den Schaumeigenschaften, beim Hautgefühl direkt nach der Anwendung zeigt Polypropylenglykol(3)myristylether (VARONIC APM^{®}) jedoch die besten Ergebnisse.

Zusammenfassend kann festgestellt werden, dass Polypropylenglykol(3)myristylether (VARONIC APM^{®}) sowohl auf die Schaumqualität als auch auf das Hautgefühl während und vor allem nach der Anwendung einen deutlich positiven Einfluss hat und den Marktstandard Polyethylenglykol(7)glycerylmonococoat (TEGOSOFT GC^{®}) teilweise sogar übertrifft.

### Beispiele 3a-k: Solubilisierende Eigenschaften

Die solubilisierenden Eigenschaften von Polypropylenglykol(3)myristylether (VARONIC APM^{®}) wurden getestet, indem ein wasserunlösliches Öl (Isopropylmyristat (TEGOSOFT M^{®})) in einer Tensidlösung, bestehend aus 11,25 Gew.-% Natriumlaurylethersulfat (Texapon NSO^{®}) und 3,75 Gew.-% Cocamidopropylbetain (TEGO Betain F 50^{®}) klar gelöst wurde. Zum Vergleich wurde das Öl in der reinen Tensidlösung ohne Solubilisator-Zusatz gelöst. Als Standards wurden die typischen Solubilisatoren Polyethylenglykol(7)glycerylmonococoat (TEGOSOFT GC^{®}) und Laureth-4 (TEGO Alkanol L 4^{®}) eingesetzt.

Tabelle 5 und Fig. 2 geben die Menge Öl an, die im jeweiligen System noch klar gelöst werden konnte. Oberhalb dieser Menge kommt es zu Trübungen.

Polypropylenglykol(3)myristylether (VARONIC APM^{®}) hat eine deutliche solubilisierende Wirkung, die den Marktstandard Polyethylenglykol(7)glycerylmonococoat (TEGOSOFT GC^{®}) übertrifft und vergleichbar ist mit dem ebenfalls gebräuchlichen Solubilisator Laureth-4 (TEGO Alkanol L 4^{®}).

**Tab. 5: Formulierungen und Ergebnisse - Solubilisierungsversuche**

| | [Gew.-] | | | | | | | | | | |
|---|---|---|---|---|---|---|---|---|---|---|---|
| Beispiel | 3a** | 3b** | 3c* | 3d* | 3e* | 3f* | 3g* | 3h* | 3i* | 3j* | 3k* |
| Texapon NSO^{®} (INCI: Sodium Laureth Sulfate) | 11,25 | 11,25 | 11,25 | 11,25 | 11,25 | 11,25 | 11,25 | 11,25 | 11,25 | 11,25 | 11,25 |
| TEGO Betain F 50^{®} (INCI: Cocamidopropyl Betaine) | 3,75 | 3,75 | 3,75 | 3,75 | 3,75 | 3,75 | 3,75 | 3,75 | 3,75 | 3,75 | 3,75 |
| TEGOSOFT M^{®} (Isopropyl Myristate) | 0,5 | 0,6 | 1,0 | 1,5 | 1,6 | 1,0 | 1,1 | 1,2 | 1,5 | 1,6 | 1,7 |
| VARONIC APM^{®} (INCI: Polypropylenglykol(3)-myristylether) | | | 0,5 | 0,5 | 0,5 | | | | | | |
| TEGOSOFT GC^{®} (INCI: PEG-7 Glyceryl Cocoate) | | | | | | 0,5 | 0,5 | 0,5 | | | |
| TEGO Alkanol L 4^{®} (INCI: Laureth-4) | | | | | | | | | 0,5 | 0,5 | 0,5 |
| Wasser | ad 100,0 | | | | | | | | | | |
| Aussehen | klar | trüb | klar | klar | trüb | klar | klar | trüb | klar | klar | trüb |

| | | | | | | | | | | | |
|---|---|---|---|---|---|---|---|---|---|---|---|
| * Vergleichsbeispiel ** Control | | | | | | | | | | | |

Zusammenfassend kann gesagt werden, dass die angegebenen Beispiele die verdickende, die pflegende und die solubilisierende Wirkung von Polypropylenglykol(3)myristylether eindeutig nachweisen, wobei die Wirksamkeit der Vergleichssubstanzen (Marktstandards) teilweise deutlich übertroffen wird.

## Patentansprüche

1. Wässrige tensidische Formulierung enthaltend
a) mindestens 10 Gew.-% mindestens eines Tensids
b) mindestens 2 Gew.-% Polypropylenglykol(3)myristylether
c) mindestens 85 Gew.-% Wasser.

2. Formulierung gemäß Anspruch 1, **dadurch gekennzeichnet, dass** Komponente a) ausgewählt ist aus den Verbindungen Natriumlaurylethersulfat und Cocamidopropylbetain und Kombinationen davon.

3. Verwendung von Polypropylenglykol(3)myristylether als Viskositätsregler, Pflegewirkstoff und Solubilisator in wässrigen tensidischen Formulierungen mit einem Wassergehalt von mindestens 75 Gew.-%.

4. Verwendung von Polypropylenglykol(3)myristylether gemäß Anspruch 3 in Abwesenheit weiterer Viskositätsregler, Pflegewirkstoffe oder Solubilisatoren.

5. Flüssige Körperreinigungsmittel enthaltend eine wässrige tensidische Formulierung nach einem der Ansprüche 1 und 2.

6. Flüssige Körperreinigungsmittel nach Anspruch 5, **dadurch gekennzeichnet, dass** das Körperreinigungsmittel ein Duschbad ist.

7. Flüssige Körperreinigungsmittel nach Anspruch 5, **dadurch gekennzeichnet, dass** das Körperreinigungsmittel ein Haarshampoo ist.

## Claims

1. Aqueous surface-active formulation comprising
a) at least 10% by weight of at least one surfactant
b) at least 2% by weight of polypropylene glycol(3) myristyl ether
c) at least 85% by weight of water.

2. Formulation according to Claim 1, **characterized in that** component a) is selected from the compounds sodium lauryl ether sulphate and cocamidopropylbetaine and combinations thereof.

3. Use of polypropylene glycol(3) myristyl ether as viscosity regulator, care active ingredient and solubilizer in aqueous surface-active formulations with a water content of at least 75% by weight.

4. Use of polypropylene glycol(3) myristyl ether according to Claim 3 in the absence of further viscosity regulators, care active ingredients or solubilizers.

5. Liquid body cleansing compositions comprising an aqueous surface-active formulation according to one of Claims 1 and 2.

6. Liquid body cleansing compositions according to Claim 5, **characterized in that** the body cleansing composition is a shower bath.

7. Liquid body cleansing compositions according to Claim 5, **characterized in that** the body cleansing composition is a hair shampoo.

## Revendications

1. Formulation tensioactive aqueuse, contenant :
a) au moins 10 % en poids d'au moins un tensioactif,
b) au moins 2 % en poids d'éther myristylique de polypropylène glycol (3),
c) au moins 85 % en poids d'eau.

2. Formulation selon la revendication 1, **caractérisée en ce que** le composant a) est choisi parmi les composés lauryléthersulfate de sodium et cocamidopropylbétaïne et leurs combinaisons.

3. Utilisation d'éther myristylique de polypropylène glycol (3) en tant que régulateur de viscosité, agent actif de soin et solubilisateur dans des formulations tensioactives aqueuses ayant une teneur en eau d'au moins 75 % en poids.

4. Utilisation d'éther myristylique de polypropylène glycol (3) selon la revendication 3 en l'absence d'autres régulateurs de viscosité, agents actifs de soin ou solubilisateurs.

5. Produit de nettoyage liquide pour le corps contenant une formulation tensioactive aqueuse selon l'une quelconque des revendications 1 et 2.

6. Produit de nettoyage liquide pour le corps selon la revendication 5, **caractérisé en ce que** le produit de nettoyage pour le corps est un gel douche.

7. Produit de nettoyage liquide pour le corps selon la revendication 5, **caractérisé en ce que** le produit de nettoyage pour le corps est un shampoing.
